Europäisches Patentamt

European Patent Office

Office européen des brevets

(19)

(11) Veröffentlichungsnummer: **0 056 489**
**B1**

(12) # EUROPÄISCHE PATENTSCHRIFT

(45) Veröffentlichungstag der Patentschrift:
22.08.84

(51) Int. Cl.³: **C 07 C 55/14, C 07 C 51/09**

(21) Anmeldenummer: **81110727.5**

(22) Anmeldetag: **23.12.81**

(54) **Verfahren zur kontinuierlichen Herstellung von Adipinsäure.**

(30) Priorität: **21.01.81 DE 3101716**

(43) Veröffentlichungstag der Anmeldung:
**28.07.82 Patentblatt 82/30**

(45) Bekanntmachung des Hinweises auf die Patenterteilung:
**22.08.84 Patentblatt 84/34**

(84) Benannte Vertragsstaaten:
**BE CH DE FR GB IT LI NL**

(56) Entgegenhaltungen:
**BE - A - 457 521**
**DE - B - 1 255 108**
**FR - A - 1 602 512**
**US - A - 2 719 166**
**US - A - 2 936 321**

**DERWENT JAPANESE PATENTS REPORT, Vol. 4, no. 29,
1965 LONDON (GB)
CHEMICAL ABSTRACTS, Band 92, Februar 1980, nr. 5,
Seite 729, Zusammenfassung 41353k COLUMBUS,
OHIO (US)**

(73) Patentinhaber: **BASF Aktiengesellschaft,
Carl-Bosch-Strasse 38, D-6700 Ludwigshafen (DE)**

(72) Erfinder: **Magnussen, Peter, Dr.,
Professor-Dillinger-Weg 25, D-6702 Bad Duerkheim (DE)**
Erfinder: **Schumacher, Volker, Dr., Auf der Hoehe 34,
D-6710 Frankenthal (DE)**
Erfinder: **Gebert, Wolfgang, Dr., Kastanienweg 18,
D-5047 Wesseling (DE)**
Erfinder: **Reitz, Heinrich, Dr., Beethovenstrasse 3,
D-6800 Mannheim (DE)**
Erfinder: **Praetorius, Werner, Dr., Dirmsteiner Weg 47,
D-6700 Ludwigshafen (DE)**

## Beschreibung

Gegenstand der Erfindung ist ein Verfahren zur kontinuierlichen Herstellung von Adipinsäure durch Verseifen von Adipinsäure-$C_1$- bis -$C_4$-Alkylestern mit Wasser bei erhöhter Temperatur in Gegenwart von stark sauren Ionenaustauschern.

Aus der japanischen Patentveröffentlichung Nr. 15166/65 ist bekannt, dass man Adipinsäure durch Hydrolyse von Adipinsäuredimethylester mit überschüssigem Wasser in Abwesenheit von Katalysatoren bei 175° C erhält. Dieses Verfahren hat den Nachteil, dass sich durch die erforderlichen hohen Temperaturen Nebenprodukte bilden, die schwer abtrennbar sind. Ferner wird in der FR-PS Nr. 1602512 erwähnt, dass die Hydrolyse von Estern mit Mineralsäuren nachteilig ist, da technisch aufwendige korrosionsfeste Vorrichtungen notwendig sind. Dort wird ausgeführt, dass die Hydrolyse mit alkalischen Mitteln zur Verfärbung und Abbau der gewünschten säure führt. Aus der FR-PS Nr. 1602512 ist auch bekannt, dass man 2-Oxopentandisäureethylester in einem Gemisch aus Alkohol und Wasser in Gegenwart von stark sauren Ionenaustauschern bei erhöhter Temperatur zu der entsprechenden Säure verseift. Das Verfahren hat den Nachteil, dass es eine Verweilzeit von 4 bis 5 h in Anspruch nimmt. Darüber hinaus eignet sich die beschriebene Arbeitsweise nicht für eine kontinuierliche technische Arbeitsweise.

Es war deshalb die technische Aufgabe gestellt, die Verseifung von Adipinsäureestern zu Adipinsäure so zu gestalten, dass keine Zersetzung der Adipinsäure stattfindet und die Reaktion auf einfache Weise kontinuierlich durchgeführt wird, ohne dass ein Abrieb des verwendeten Ionenaustauschers stattfindet.

Diese Aufgabe wird gelöst in einem Verfahren zur kontinuierlichen Herstellung von Adipinsäure durch Verseifen von Adipinsäure-$C_1$- bis -$C_4$-Alkylestern mit Wasser bei erhöhter Temperatur in Gegenwart von stark sauren Ionenaustauschern, wobei man Adipinsäure-$C_1$- bis -$C_4$-Alkylester und Wasser im Überschuss dem mittleren Teil einer Kolonne zuführt, unterhalb der Zuführungsstelle im Verlauf der Kolonne an mehreren Böden Reaktionsgemisch entnimmt, über einen stark sauren Ionenaustauscher leitet und das so behandelte Gemisch zurückführt, am Kopf der Kolonne Alkanole oder Alkanol/Wasser-Gemische und am unteren Ende der Kolonne eine wässerige Lösung von Adipinsäure entnimmt.

Das neue Verfahren hat den Vorteil, dass es technisch einfach in kontinuierlicher Arbeitsweise durchführbar ist und die Ionenaustauscher auch ohne Unterbrechung des Betriebes ausgetauscht werden können. Ferner hat das neue Verfahren den Vorteil, dass keine Zersetzung der Adipinsäure eintritt. Schliesslich hat das neue Verfahren den Vorteil, dass es mit kürzerer Verweilzeit verläuft und ein Abrieb der Ionenaustauscher nicht zu befürchten ist.

Als Ausgangsstoffe verwendet man Adipinsäure-$C_1$- bis -$C_4$-Alkylester. Geeignete Ester sind beispielsweise Adipinsäuredimethyl-, Adipinsäurediethyl-, Adipinsäuredipropyl- oder Adipinsäuredibutylester. Besondere technische Bedeutung hat Adipinsäuredimethylester erlangt.

Die Hydrolyse wird mit Wasser im Überschuss durchgeführt, d.h. man wendet je Mol Adipinsäureester mehr als 2 mol Wasser an. Die Wassermenge richtet sich nach der gewünschten Konzentration der herzustellenden Adipinsäurelösung. Vorteilhaft wendet man je Mol Adipinsäureester 3,5 bis 40 insbesondere 15 bis 20 mol Wasser an.

Adipinsäureester und Wasser werden vorteilhaft vorgemischt dem mittleren Teil einer Kolonne zugeführt. Zweckmässig verwendet man Kolonnen, die zumindest unterhalb der Zuführungsstelle in den jeweiligen praktischen Böden einen Flüssigkeitsstand haben. Geeignete Kolonnen sind beispielsweise Glockenbödenkolonnen oder Ventilbodenkolonnen. Unterhalb der Zuführungsstelle von Adipinsäureester und Wasser hat die Kolonne vorteilhaft 10 bis 35 praktische Böden und oberhalb der Zuführungsstelle vorteilhaft 15 bis 20 praktische Böden. Für den Verstärkungsteil oberhalb der Zuführungsstelle sind Kolonnenausführungen geeignet, die zur Trennung von Gemischen aus Alkanolen, Wasser und Ester geeignet sind. Unterhalb der Zuführungsstelle des Gemisches aus Adipinsäureester und Wasser entnimmt man vorteilhaft jedem praktischen Boden der Kolonne Reaktionsgemisch. Das Reaktionsgemisch besteht im wesentlichen aus einem Gemisch aus Adipinsäureester, Wasser, Adipinsäure und Alkanolen. Das Gemisch leitet man über einen stark sauren Ionenaustauscher und führt es wieder in die Kolonne zurück. Zweckmässig führt man das behandelte Gemisch auf den gleichen Boden oder ein bis zwei Böden über der Entnahmestelle wieder der Kolonne zu. Besonders bewährt hat es sich, das behandelte Gemisch wieder auf den gleichen Boden zurückzuführen. Es hat sich auch bewährt, wenn man Reaktionsgemisch nur jedem dritten bis fünften praktischen Boden der Kolonne entnimmt und über den stark sauren Ionenaustauscher leitet. Vorteilhaft führt man die 5- bis 30fache, insbesondere 15- bis 20fache Menge des stündlichen Kolonnendurchsatzes an jeder Entnahmestelle über den stark sauren Ionenaustauscher.

In der Kolonne wird somit unterhalb der Zuführungsstelle von Adipinsäureester und Wasser Adipinsäureester in fortschreitendem Masse hydrolysiert und die abgespalteten Alkanole abdestilliert, wobei oberhalb der Zuführungsstelle eine Abtrennung der Alkanole eintritt. Man entnimmt somit am Kopf der Kolonne die jeweils abgespalteten Alkanole oder deren Gemische mit Wasser und am unteren Ende der Kolonne eine wässerige Lösung von Adipinsäure, z.B. eine 20 bis 85 gew.%ige wässerige Lösung von Adipinsäure.

Im Kolonnenteil unterhalb der Zuführungsstelle hält man vorteilhaft eine Temperatur von 80 bis 130, insbesondere 90 bis 120° C, ein. Diese Temperaturen gelten auch für das im Kreislauf geführte Reaktionsgemisch. Es versteht sich, dass sich die Temperaturen auch nach dem Siedepunkt der abzuspaltenden Alkanole richten. Dies gilt auch für den Verstärkungsteil der Kolonne oberhalb der Zuführungsstelle.

Geeignete stark saure Ionenaustauscher sind z.B. vernetzte Polystyrole mit sauren Gruppen, insbesondere Sulfonsäuregruppen. Besonders bewährt haben sich Styrol/Divinylbenzol-Copolymerisate mit Sulfonsäuregruppen. Darüber hinaus sind auch andere stark saure und wasserunlösliche Feststoffe wie Zeolithe verwendbar. Je Liter Reaktionsgemisch und Stunde verwendet man in den einzelnen Kreisläufen vorteilhaft 0,1 bis 10 l stark saure Ionenaustauscher. Je geringer die Austauschermenge gewählt wird, um so höher ist der Dampfverbrauch bei einem bestimmten angestrebten Umsatz zu Adipinsäure.

Aus der am unteren Ende der Kolonne entnommenen wässerigen Adipinsäurelösung wird Adipinsäure zweckmässig durch Kristallisation z.B. Vakuumkristallisation abgetrennt. Die so erhaltene Mutterlauge, die z.B. 2 bis 6 Gew.-% Adipinsäure und Verunreinigungen enthält, wird vorzugsweise im Kreis geführt, d.h. wieder mit frischem Adipinsäureester der Kolonne zugeführt. Es versteht sich, dass die fehlende Wassermenge entsprechend ergänzt wird. Vor der Wiederverwendung der bei der Kristallisation anfallenden Mutterlauge wird sie zweckmässig behandelt z.B. durch Extraktion mit Adipinsäureester oder insbesondere durch Leiten über Aktivkohle.

Die erfindungsgemäss hergestellte Adipinsäure eignet sich zur Herstellung von Hexamethylendiammoniumadipat.

Das Verfahren nach der Erfindung sei in folgenden Beispielen veranschaulicht.

*Vergleichsbeispiel:*

350 g Adipinsäuredimethylester und 700 g Wasser wurden in einem von Stickstoff durchströmten Rührautoklaven zur Reaktion gebracht. Die Menge des Stickstoffs wurde so eingestellt, dass bei dem jeweiligen Druck etwa 100 g/h Wasser/Methanol-Gemisch ausgetragen wurde. Während bei 182° C im Reaktionsgemisch nach 1,5 h nur 22% Umsatz zu Adipinsäure gefunden wurden, erreichte der Umsatz bei 227° C 75%. Das bei 227° C erhaltene Produkt war jedoch deutlich gelb verfärbt.

Ein Zusatz von 10% Zeolith ergab bei 180° C nach 1,5 h einen Umsatz von 42% zu Adipinsäure.

*Beispiel 1:*

Die Verseifung von Adipinsäuredimethylester wurde in einer 80-cm-Glockenbodenkolonne durchgeführt. Die Glockenbodenkolonne hatte 10 praktische Böden. Auf dem ersten Boden wurden 150 g Adipinsäuredimethylester und 300 g Wasser/h zudosiert. Von jedem der zehn Böden im Abtriebsteil unterhalb der Zuführungsstelle wurden 2 l/h Reaktionsgemisch entnommen, über ein Festbett mit 50 ml Ionenaustauscher aus vernetztem Polystyrol mit Sulfonsäuregruppen geleitet und wieder auf denselben Boden zurückgeführt. Die Reaktion erfolgte drucklos bei einer Temperatur von 95 bis 105° C. Im Sumpf der Kolonne fiel eine wässerige Lösung mit 31 Gew.-% Adipinsäure und 1 Gew.-% Adipinsäuremonomethylester an. Am Kopf der Kolonne wurden 69 ml Methanol/h abgenommen. Der Sumpfaustrag wurde in einen Kühlungskristaller gepumpt. Die Adipinsäure wurde auf einem Bandfilter von der Mutterlauge getrennt. Die Mutterlauge wurde mit Wasser ergänzt und zur Reaktionskolonne zurückgeleitet.

*Beispiel 2:*

Die Verseifung von Adipinsäuredimethylester wurde in einer technischen Reaktionskolonne durchgeführt, bestehend aus einer Glockenbodenkolonne mit 30 praktischen Böden und einer aufgesetzten Füllkörperkolonne. Auf den ersten Boden der Glockenbodenkolonne wurden 12 kg/h Adipinsäuredimethylester und 26 kg/h Wasser bzw. Mutterlauge zugeführt. Von jedem dritten der dreissig Böden im Antriebsteil wurden 600 kg/h Reaktionsgemisch entnommen, dieses jeweils über ein Festbett mit saurem Ionenaustauscher (vernetztes Polystyrol mit Sulfonsäuregruppen) gepumpt und auf denselben Boden, von dem es entnommen wurde, zurückgeführt.

Die wässerige Adipinsäurelösung im Sumpf enthielt nur noch 0,1 Gew.-% Adipinsäuremonomethylester und keinen Adipinsäuredimethylester. Der Sumpfaustrag wurde in einem Vakuumkristallisator geleitet und die Adipinsäure in einer Zentrifuge von der Mutterlauge getrennt. Die Mutterlauge, die noch 5,5 Gew.-% Adipinsäure enthält, wurde mit Wasser ergänzt und wieder der Reaktionskolonne zugeleitet.

*Beispiel 3:*

Die Mutterlauge nach Beispiel 1 wurde in einem Mischer-Scheider mit Adipinsäuredimethylester im Volumenverhältnis 10:1 bei 40° C extrahiert. Die UV-Zahl der Mutterlauge, die ein Mass für die Konzentration von Verunreinigungen in der Mutterlauge darstellt, ging dabei um 50% zurück.

*Beispiel 4:*

Die Mutterlauge nach Beispiel 1 wurde bei 30° C über ein Festbett mit 50 g aschefreier Aktivkohle geleitet. Die UV-Zahl der Mutterlauge sank dabei um 40% ab.

**Patentansprüche**

1. Verfahren zur kontinuierlichen Herstellung von Adipinsäure durch Verseifen von Adipinsäure-$C_1$- bis -$C_4$-Alkylestern mit Wasser bei erhöhter Temperatur in Gegenwart von stark sauren Ionenaustauschern, dadurch gekennzeichnet, dass man Adipinsäure-$C_1$- bis -$C_4$-Alkylester und Wasser im Überschuss dem mittleren Teil einer Kolonne zuführt, unterhalb der Zuführungsstelle im Verlauf der Kolonne an mehreren Böden Reaktionsgemisch entnimmt, über einen stark sauren Ionenaustauscher leitet und das so behandelte Gemisch zurückführt, am Kopf der Kolonne Alkanole oder Alkanol/Wasser-Gemische und am unteren Ende der Kolonne eine wässerige Lösung von Adipinsäure entnimmt.

2. Verfahren nach Anspruch 1, dadurch gekennzeichnet, dass man je Mol Adipinsäureester 3,5 bis 40 mol Wasser anwendet.

3. Verfahren nach den Ansprüchen 1 und 2, dadurch gekennzeichnet, dass man in der Kolonne unterhalb der Zuführungsstelle von Adipinsäureester und Wasser eine Temperatur von 80 bis 130° C einhält.

4. Verfahren nach den Ansprüchen 1 bis 3, dadurch gekennzeichnet, dass man unterhalb der Zuführungsstelle von Adipinsäureester und Wasser an jedem 3. bis 5. praktischen Boden Reaktionsgemisch entnimmt und über den stark sauren Ionenaustauscher leitet.

5. Verfahren nach den Ansprüchen 1 bis 4, dadurch gekennzeichnet, dass man das entnommene Reaktionsgemisch jeweils auf den gleichen Boden in die Kolonne zurückführt.

6. Verfahren nach den Ansprüchen 1 bis 5, dadurch gekennzeichnet, dass man jeweils die 5- bis 30fache Menge des Kolonnendurchsatzes über den stark sauren Ionenaustauscher leitet.

7. Verfahren nach den Ansprüchen 1 bis 6, dadurch gekennzeichnet, dass man aus der erhaltenen wässerigen Adipinsäurelösung Adipinsäure auskristallisiert und die so erhaltene Mutterlauge in den Zulauf zur Kolonne zurückführt.

8. Verfahren nach den Ansprüchen 1 bis 7, dadurch gekennzeichnet, dass die Mutterlauge nach Abtrennen der Adipinsäure mit Adipinsäureester extrahiert oder mit Aktivkohle behandelt wird.

## Claims

1. A process for the continuous preparation of adipic acid by hydrolysis of $C_1$-$C_4$-alkyl adipates with water at elevated temperature in the presence of a strongly acidic ion exchanger, wherein the $C_1$-$C_4$-alkyl adipates and excess water are fed to the middle section of a column, reaction mixture is taken off at a plurality of trays of the column below the feed point and passed over a strongly acidic ion exchanger, the so-treated mixture is recycled, alkanols or alkanol/water mixtures are taken off at the top of the column and an aqueous solution of adipic acid is taken off at the bottom of the column.

2. A process as claimed in Claim 1, wherein from 3.5 to 40 mol of water are employed per mole of adipic acid ester.

3. A process as claimed in Claims 1 and 2, wherein the temperature in the column, below the feed point of adipic acid ester and water, is kept at from 80 to 130° C.

4. A process as claimed in Claims 1 to 3, wherein reaction mixture is taken off at every 3rd to 5th tray below the feed point of adipic acid ester and water, and is passed over the strongly acidic ion exchanger.

5. A process as claimed in Claims 1 to 4, wherein each stream of reaction mixture taken off is recycled to the same tray in the column.

6. A process as claimed in Claims 1 to 5, wherein the amount passed over the strongly acidic ion exchanger from each take-off point is from 5 to 30 times as great as the through put of the column.

7. A process as claimed in Claims 1 to 6, wherein the adipic acid is crystallized out of the resulting aqueous adipic acid solution, and the mother liquor thus obtained is recycled to the feed to the column.

8. A process as claimed in Claims 1 to 7, wherein the mother liquor obtained after separation of the adipic acid is extracted with adipic acid ester or treated with active charcoal.

## Revendications

1. Procédé de préparation continue d'acide adipique par saponification d'esters alkyliques (en $C_1$ à $C_4$) d'acide adipique avec de l'eau, à température élevée, en présence d'échangeurs d'ions fortement acides, caractérisé par le fait qu'on amène l'ester alkylique (en $C_1$ à $C_4$) d'acide adipique et de l'eau, en excès, à la partie médiane d'une colonne, par le fait qu'on prélève du mélange de réaction, sur plusieurs plateaux, en dessous de la zone d'amenée dans le courant de la colonne, qu'on l'amène sur un échangeur d'ions fortement acide et qu'on recycle le mélange ainsi traité, qu'on prélève en tête de colonne des alcanols ou des mélanges alcanol/eau et, à l'extrémité inférieure de la colonne, une solution aqueuse d'acide adipique.

2. Procédé selon la revendication 1, caractérisé par le fait qu'on utilise 3,5 à 40 mol d'eau par mole d'ester adipique.

3. Procédé selon les revendications 1 et 2, caractérisé par le fait qu'on maintient dans la colonne, sous la zone d'amenée de l'ester adipique et de l'eau, une température de 80 à 130° C.

4. Procédé selon l'une des revendications 1 à 3, caractérisé par le fait que, sous la zone d'amenée de l'ester adipique et de l'eau, on prélève du mélange de réaction tous les 3 à 5 plateaux et on l'envoie sur l'échangeur d'ions fortement acide.

5. Procédé selon l'une des revendications 1 à 4, caractérisé par le fait que l'on recycle le mélange de réaction prélevé sur le même plateau de la colonne.

6. Procédé selon l'une des revendications 1 à 5, caractérisé par le fait que l'on envoie chaque fois sur l'échangeur d'ions fortement acide une quantité correspondant à 5 à 30 fois le débit de la colonne.

7. Procédé selon l'une des revendications 1 à 6, caractérisé par le fait que, de la solution aqueuse d'acide adipique obtenue, on fait cristalliser de l'acide adipique et qu'on recycle dans le courant de la colonne l'eau mère ainsi obtenue.

8. Procédé selon l'une des revendications 1 à 7, caractérisé par le fait que l'eau mère, après séparation de l'acide adipique, est extraite avec de l'ester adipique ou traitée avec du charbon actif.